# EUROPEAN PATENT APPLICATION

(11) **EP 0 925 790 A1**
(43) Date of publication of application: **30.06.1999**
(21) Application number: 97310213.0
(22) Date of filing: 17.12.1997
(51) Int. Cl.: A61K 33/34, A61K 33/32

(54) **Vitamin and mineral combinations for the treatment of syndrome X and non-insulin dependent diabetes (NIDDM)**

(71) Applicant: Lalvani, Kartar, Dr., Wembley, Middlesex HA0 1NU (GB); Beckett, Arnold Heyworth, Upper Norwood, London SE19 2UN (GB); Taylor, Robert P., London W11 1EP (GB)
(72) Inventor: Lalvani, Kartar, Dr., Wembley, Middlesex HA0 1NU (GB); Beckett, Arnold Heyworth, Upper Norwood, London SE19 2UN (GB); Taylor, Robert P., London W11 1EP (GB)

(57) **Abstract**

This invention relates to the use of novel combinations of vitamins and minerals to give a product which has the action of either a direct reaction with the insulin receptor in NIDDM patients to facilitate the receptor's interaction with the circulating insulin in the human body, or acts to increase the action of appropriate enzymes in the NIDDM patients to provide *in vivo* the ingredients required to restore the functioning of the insulin receptor site.

The product of the invention, which is composed of mixtures of specific metallic cations and non-metallic elements to which certain vitamins may be added, not only acts to slow down the development of NIDDM when Syndrome X has started, but also can treat successfully those who have developed NIDDM and help those of more advanced NIDDM when the insulin production is deteriorating.

## Description

Non-insulin dependent diabetes (NIDDM), also called type II diabetes mellitus, is a chronic disorder characterised by an elevated glucose concentration in the blood due to the diminished effectiveness of insulin.

At the early stages of development, it can be identified by impaired glucose tolerance and hyperinsulinaemia as the body seeks to correct for the diminished effectiveness of the insulin produced. Later development NIDDM can be complicated by impaired insulin secretion.

Recently it has been established that the combination of insulin resistance and the compensatory hyperinsulinaemia predisposes to the cluster of metabolic disorders known as Syndrome X which occurs before NIDDM has been diagnosed as such. Syndrome X includes obesity, hypertension, high triglyceride levels and low high density lipoprotein (HDL) cholesterol concentrations, insulin resistance and elevated insulin levels. This constitutes an important risk factor for atherosclerotic vascular disease.

Diabetes, of which NIDDM represents 85 to 90% of the total, is a serious and rising global epidemic. Since 1987, the number of sufferers worldwide has tripled and now represents about 6 per cent of the world's adult population. In the USA alone, the cost to the country is now considered to be about $100 billion. The complications caused by diabetes present the greatest burden, and are responsible for the following; more than half of all leg amputations; the leading cause of kidney disease in USA and Europe; the major cause of blindness in the middle-aged in Europe; a doubling of the risk of heart attack or stroke; the major cause of impotence in men of all ages.

The public awareness of diabetes is low; at least half of all people with diabetes are unaware of their condition and in some countries it is estimated that the proportion of undiagnosed cases may be as high as 80%.

In NIDDM, the body is unable to use properly the insulin it produces. The insulin receptors with which the insulin must react to give its normal affects, do not respond correctly or are inadequate for appropriate functioning.

The treatment with synthetic drugs currently comprises:

### Sulphonyl Ureas

Tolbutamide
Chlorpropamide
Tolazamide
Glipizide
Gliclazide

### Biguanides

Metformin
Phenformin

These therapies emphasise the treatment of the symptoms of NIDDM rather than dealing with the primary cause.

The present invention relates to the use of appropriate combinations of vitamins and minerals to give a product with a balance of ingredient which have one or both of the following actions;
1. A direct reaction with the insulin receptor in NIDDM patients, to facilitate its interaction with the circulating insulin in the human body, to thus promote the typical insulin responses to restore the normal functions of insulin.
2. Act to increase the action of appropriate enzymes in the NIDDM patients to provide *in vivo* the ingredients which are incorporated into or become part of the insulin receptor site to thus restore its functioning and make the patient react to the normal or deteriorating supply of insulin *in vivo*.

Thus the products of the invention not only act to slow down the development of NIDDM when Syndrome X has started, but also can treat successfully those who have developed NIDDM and help those of more advanced NIDDM when the insulin production is deteriorating.

The products of the invention are composed of mixtures of one or more of the metals chromium, copper, zinc, iron, manganese, magnesium as cations and the non metal elements iodine, selenium as anions, in various proportions. To these components vitamins A, C, D and folic acid may be added to form the final product.

The proportion of various ingredient by elemental weight, relative to chromium considered as unity, are in the ranges:

For the metal cations of Cu⁺⁺ (0-200), Fe⁺⁺ (0-4,000), Zn⁺⁺ (0-1,200), Mn⁺⁺ (0-600) and Mg⁺⁺ (0-20,000) in the form of their oxides or their salts involving the anions chloride, bromide, sulphate, carbonate, phosphate, lower chain (1-6) aliphatic in their branched and unbranched forms as anions, and also their hydroxy derivatives, plus the non-metals selenium and iodine in the form of Se^{2-,4-,6-} (0-17) and I⁻ (0-50) as anions in salts with sodium and potassium cations, and also for vitamins of the B group in the proportions relative to chromium of B1 (0-3,000), B2 (0-4,000), B3 (0-3,000), B6 (0-6,000), B12 (0-2).

To the above the following may be optionally added, in proportions relative to that of chromium; Vitamin A as retinol equivalents (0-200), Vitamin C (0-60,000), vitamin D (0-0.5), vitamin E (0-10,000), folic acid (0-40), pantothenic acid (0-4,000), biotin (0-10).

If chromium is omitted from the mixture then the mean of the range of one of the other metals may be used as the standard.

A typical product is constituted as follows:

### Example 1

| | |
|---|---|
| Chromium | 200 mcg |
| Selenium | 100 mcg |
| Copper | 1 mg |
| Zinc | 15 mg |
| Iron | 8 mg |
| Manganese | 2 mg |
| Iodine | 100 mcg |
| Magnesium | 100 mg |
| Thiamin (Vitamin B1) | 15 mg |
| Riboflavin (Vitamin B2) | 5 mg |
| Niacin (Vitamin B3) | 45 mg |
| Vitamin B6 | 25 mg |
| Vitamin B12 | 9 mcg |
| Vitamin A | 700 mcg |
| Vitamin C | 120 mg |
| Vitamin D | 5 mcg |
| Folic Acid | 500 mcg |
| Vitamin E | 30 mg |
| Biotin | 200 mcg |
| Pantothenic Acid | 10 mg |

### Example 2

| | |
|---|---|
| Chromium | 500 mcg |
| Selenium | 200 mcg |
| Copper | 5 mg |
| Zinc | 15 mg |
| Iron | 14 mg |
| Manganese | 20 mg |
| Iodine | 500 mcg |
| Magnesium | 100 mg |
| Thiamin (Vitamin B1) | 75 mg |
| Riboflavin (Vitamin B2) | 75 mg |
| Niacin (Vitamin B3) | 75 mg |
| Vitamin B6 | 50 mg |
| Vitamin B12 | 30 mcg |
| Folic Acid | 500 mcg |

The above combinations may be combined with herbal materials, the preferred being standardised propolis.

These examples are given by way of illustration only, and are not constructed as limiting the invention in scope or in spirit, as many modifications will be apparent from disclosure to those in this art.

The invention involves the ingredients in any pharmaceutical form, for example, tablet, capsule, liquid, patch, injection, or solution.

## Claims

1. Products for the treatment in animals and humans of Syndrome X and Non-Insulin Dependent Diabetes comprising chromium (elemental 5-500mcg), and copper (elemental 0.2-10mg) as cations.

2. Products as claimed in claim 1, with the addition of one or more B group vitamins.

3. Products as claimed in claim 1 with the addition of 1-400mcg selenium as an anion.

4. Products as claimed in claim 2 with the addition of 1-400mcg selenium as an anion.

5. Product as claimed in claim 3 with the addition of 1-200mg iron as a cation.

6. Product as claimed in claim 4 with the addition of 1-200mg iron as a cation.

7. Products as claimed in claim 1 with the addition of 1-60mg zinc as a cation.

8. Products as claimed in claim 2 with the addition of 1-60mg zinc as a cation.

9. Products as claimed in claim 3 with the addition of 1-60mg zinc as a cation.

10. Products as claimed in claim 4 with the addition of 1-60mg zinc as a cation.

11. Products as claimed in claim 5 with the addition of 1-60mg zinc as a cation.

12. Products as claimed in claim 6 with the addition of 1-60mg zinc as a cation.

13. Products as claimed in claim 1 with the addition of 0.5-60mg manganese as a cation.

14. Products as claimed in claim 2 with the addition of 0.5-60mg manganese as a cation.

15. Products as claimed in claim 4 with the addition of 0.5-60mg manganese as a cation.

16. Products as claimed in claim 6 with the addition of 0.5-60mg manganese as a cation.

17. Products as claimed in claim 8 with the addition of 0.5-60mg manganese as a cation.

18. Products as claimed in claim 12 with the addition of 0.5-60mg manganese as a cation.

19. Products for the treatment in animals and humans of Syndrome X and Non-Insulin Dependent Diabetes comprising zinc (elemental 1-60mg) and manganese (elemental 0.5-60mg) as cations.

20. Products as claimed in claim 19 with the addition of 1-400mcg selenium as an anion.

21. Product as claimed in claim 19 with the addition of 1-200mg iron as a cation.

22. Product as claimed in claim 20 with the addition of 1-200mg iron as a cation.

23. Product as claimed in claim 22 with the addition of one or more B group vitamins.
